# EUROPEAN PATENT SPECIFICATION

(11) **EP 3 297 687 B1**
(45) Date of publication and mention of the grant of the patent: **21.10.2026**
(21) Application number: 16742020.7
(22) Date of filing: 20.05.2016
(51) Int. Cl.: A61L 2/202, A61L 2/183, A61L 9/14

(54) **DECONTAMINATION DEVICE AND METHOD FOR MEDICAL INSTRUMENTS**
DEKONTAMINATIONSVORRICHTUNG UND VERFAHREN FÜR MEDIZINISCHE INSTRUMENTE
DISPOSITIF DE DÉCONTAMINATION ET PROCÉDÉ ASSOCIÉ POUR INSTRUMENTS MÉDICAUX

(30) Priority: 21.05.2015 NL 2014837
(43) Date of publication of application: 28.03.2018
(73) Proprietor: Log10 B.V., 5642 JC Eindhoven (NL)
(72) Inventor: VAN DEN HOUDT, Andreas Adrianus Lambertus, 5642 JC Eindhoven (NL); VERHOEVEN, Franciscus Maria, 5642 JC Eindhoven (NL); PESSERS, Paul Herman Maria, 5642 JC Eindhoven (NL)
(74) Representative: V.O.
(86) International application number: PCT/NL2016/050360
(87) International publication number: WO 2016/186502

(56) References cited:
- EP-A1- 1 787 662
- EP-A1- 2 292 340
- EP-A2- 1 787 731
- EP-B1- 1 444 992
- WO-A1-2005/070205
- WO-A1-2007/000639
- WO-A1-2007/045943
- WO-A1-2009/030599
- CN-U- 203 635 580
- DE-A1- 2 339 517
- US-A1- 2002 153 021

## Description

### FIELD OF THE INVENTION

The present invention relates to a decontamination device and method for medical instruments. More in particular, the present invention relates to a device for disinfecting and/or sterilizing medical instruments, such as dental instruments.

### BACKGROUND TO THE INVENTION

Medical instruments that have been used in a medical procedure are often contaminated with body fluids, tissue, micro-organisms or the like. The contaminated instruments can only be used again after decontamination to avoid cross-contamination from one patient to another. Decontamination can include one or more of rinsing, washing, drying, disinfecting and sterilizing. Normally these steps are carried out by trained personnel and/or dedicated machinery.

EP1787731A2 discloses a washing apparatus, in particular for heat-disinfection, comprising a plurality of washer machines, which are positioned aligned along an axis of alignment, and a movement device to move an object-bearing container parallel to the axis of alignment. The movement device is provided with a trolley to transport the container and having sliding means guided by a guide element which extends parallel to the axis of alignment. The movement device is also provided with movement means to move the trolley parallel to the guide element. The washing apparatus also comprises a frame, disposed along the axis of alignment and made so as to define a plurality of housing zones in which the washer machines are positioned. The movement means is associated at least partly with the frame.

### SUMMARY OF THE INVENTION

It is an object of the invention to provide a decontamination device and a method that render the task of decontaminating a medical instrument less cumbersome, less time-consuming and/or less expensive.

Thereto according to the invention is provided a decontamination device for medical instruments as defined in claim 1. The device includes a reception unit arranged for receiving a to be treated medical instrument loaded in a cassette wherein the cassette includes a first portion and a second portion that can be closed against each other for enclosing the medical instrument. The device includes a washing unit arranged for washing and/or drying the instrument in the cassette. The device includes a disinfection unit arranged for disinfecting and/or sterilizing the instrument in the cassette, e.g. the washed and/or dried instrument. The device includes a wrapping unit arranged for sealing the cassette containing the disinfected and/or sterilized instrument. The device includes a delivery unit arranged for presenting the cassette containing the disinfected and/or sterilized instrument to be taken out of the decontamination device.

This provides the advantage that all functionality for cleaning a medical instrument is present within one single device. Thus, cleaning the medical instrument is easier, faster, requires less handling time and requires no human interaction during the decontamination process.

Optionally, the wrapping unit is arranged for providing the disinfected instrument in a holder. The instrument in the holder can be hermetically sealed from ambient air. The delivery unit can then be arranged for presenting the, e.g. hermetically sealed, holder including the decontaminated instrument to be taken out of the decontamination device. This provides the advantage that the instrument, once disinfected, is provided in the holder protected against contamination. The holder can then be opened prior to use of the disinfected instrument. Cleanliness of the instrument can hence be guaranteed to a great extent.

Optionally, the decontamination device includes a control unit arranged for controlling the washing unit, the disinfection unit and optionally the wrapping unit, for automatically washing and/or drying and disinfecting the instrument and optionally automatically providing the disinfected instrument in the holder hermetically sealed from ambient air. Hence the entire cleaning of the instrument can be automated. Thus, an operator only needs to enter the to be treated instrument into the reception unit and to retrieve the disinfected instrument from the delivery unit. No manual handling of the partially decontaminated instrument is required.

Optionally, the reception unit and the delivery unit are one and the same. This provides the advantage that the disinfected instrument can be retrieved at the same unit where it was entered into the device. This may also allow for designing the device to be compact. It will be appreciated that it is possible that the washing unit, the disinfecting unit and optionally the wrapping unit share the same space.

Optionally, at least the reception unit, the disinfection unit, and the delivery unit are consecutive stations within the device. This provides the advantage that the device can be designed as a feed-though device. This allows a part of the device that comes into contact with contaminated instruments to be physically separated, or at least at a distance, from a part of the device that presents the decontaminated instruments. Hence, cross-contamination may be prevented. It is for instance possible that the reception unit is positioned in a first room and the delivery unit is in a different second room, the decontamination device passing through a divider, such as a wall, separating the first and second rooms. Hence it is possible to provide a physical barrier between a contaminated side of the decontamination device and a decontaminated side of the decontamination device.

Optionally, the washing unit is a consecutive station within the device, e.g. located between the reception unit and the disinfection unit. Hence, disinfecting can be performed after washing. Optionally, the wrapping unit is a consecutive station within the device, e.g. located between the disinfection unit and the delivery unit.

The disinfection unit is arranged for disinfecting and/or sterilizing the instrument. It will be appreciated that depending on the circumstances it may suffice to disinfect an instrument, wherein a large proportion of microorganisms is killed, although not all microorganisms are necessarily killed. In other cases it is preferred to sterilize the instrument, wherein substantially all microorganisms are killed.

Optionally, the disinfection unit is arranged for treating the instrument with a disinfectant, such as ozone. Optionally the disinfection unit is arranged for treating the instrument with a gaseous disinfectant, such as gaseous ozone. The ozone can be used for disinfection or sterilizing the instrument.

Optionally, the washing unit is arranged for washing debris off the instrument. Hence, debris, such as tissue, can be rinsed off the instrument. Washing off debris can precede a further washing step.

Optionally, the washing unit includes at least one nozzle to spray a jet or mist of a cleaning fluid directly onto the instrument. Hence, the instrument can be washed effectively. The cleaning fluid can be water, a detergent or a combination thereof. Optionally, the decontamination device, e.g. the washing unit, is arranged for washing the instrument using a liquid, such as water, containing a disinfectant, such as ozone. Washing with a liquid containing the disinfectant, e.g. ozone, can already provide a level of disinfection. Washing with a liquid containing the disinfectant can follow an earlier washing step such as washing off debris and/or washing with water and/or detergent.

Optionally, the washing unit includes at least one nozzle to spray a jet or mist of a drying gas directly onto the instrument. Hence drying of the washed instrument can be performed effectively.

Optionally, the decontamination device, e.g. the washing unit, is arranged for drying the instrument using a gas containing a disinfectant, such as ozone. Drying with a gas containing the disinfectant, e.g. ozone, can already provide a level of disinfection. It will be appreciated that using ozone provides the advantage that it can easily be used both as component in the washing liquid, and in gaseous form, e.g. for drying and in the disinfection unit. When the washing unit is arranged for drying and located between the reception unit and the disinfection unit, disinfecting can be performed after washing and drying. More in general disinfecting can be performed after drying.

Optionally, the wrapping unit is arranged for packaging the disinfected instrument in a hermetically sealed holder. The hermetically sealed holder can be a bag, a box, a container, or the like. This provides the advantage that the instrument, once disinfected, is protected against contamination microbial growth in the hermetically sealed holder. The holder can then be opened prior to use of the disinfected instrument. Cleanliness of the instrument can hence be guaranteed to a great extent. An indication of an expiry date can be placed on the hermetically sealed holder.

The decontamination device is arranged for holding the instrument in the cassette. Hence, the instrument can be treated in the device while positioned in the cassette. Using the cassette can greatly improve the ease of handling different instruments. It is possible that the cassette is arranged for holding a plurality of instruments. It is also possible that the cassette is arranged for holding a specific instrument or a specific type of instrument. It is also possible that the cassette is arranged for holding one or more of different types of instruments.

The reception unit is arranged for receiving the to be treated medical instrument loaded in the cassette. Hence, the instrument can be loaded in the cassette and the cassette can be inserted into the reception unit. The washing unit is arranged for washing and/or drying the instrument in the cassette. Hence, the instrument need not be taken out of the cassette for being washed and/or dried. This greatly simplifies handling of the instrument inside the washing unit. The disinfection unit is arranged for disinfecting and/or sterilizing the instrument in the cassette. Hence, the instrument need not be taken out of the cassette for being disinfected or sterilized. This greatly simplifies handling of the instrument inside the disinfection unit. Optionally, the wrapping unit is arranged for hermetically sealing the cassette containing the disinfected instrument. Thus the cassette can also serve as the holder. The delivery unit is arranged for presenting the cassette containing the disinfected and/or sterilized instrument to be taken out of the decontamination device. Hence, the disinfected instrument can be taken out of the decontamination device while remaining in the cassette. This greatly reduces the risk of the instrument becoming contaminated while or after taking the disinfected instrument out of the delivery unit.

The cassette includes the first portion and the second portion that can be closed against each other for enclosing the instrument. The first portion can e.g. be a tray and the second portion can e.g. be a lid. This provides the advantage that the instrument, or a plurality of instruments, can easily be loaded into the cassette. The cassette can include one or more openings for allowing washing liquids, drying gases and/or disinfectants to pass into and/or out of the cassette.

Optionally, the wrapping unit is arranged for hermetically closing the first portion against the second portion. Thereto the wrapping unit can e.g. by provide a seal, such as a self-adhesive ribbon or glue, at the interface of the first and second portions. Hence the cassette can easily be hermetically closed. The wrapping unit can also be arranged for hermetically closing the one or more openings of the cassette.

The cassette can be reusable. It is also possible that the cassette is a disposable, intended for single use.

Optionally, the decontamination device includes a transportation unit arranged for transporting the instrument and/or the cassette from the reception unit to the disinfection unit, possibly via the washing unit. Optionally, the transportation unit is arranged for transporting the instrument and/or the cassette from the disinfection unit to the delivery unit, possibly via the wrapping unit. It will be appreciated that the transportation unit may include one or more transportation sections for transporting the instrument and/or cassette from one unit to another.

Optionally the cassette has one or more interface locations for interfacing with the transportation unit.

Optionally, the decontamination device includes a labelling unit arranged for providing the instrument and/or the cassette with a label representative of the performed decontamination. The label can e.g. be a sticker or can be printed onto the instrument and/or the cassette. Hence it is possible to determine the decontamination performed on the instrument from the label. The label can include information on the performed decontamination, e.g. a code representative of performed process steps. The label can also include information representative of a location in a database where information on the performed decontamination is stored. The label can include information representative of a date and time of decontamination.

Optionally, the instrument is a dental instrument. The dental instruments can e.g. be scalpels, syringes, scopes , mirrors, drills, burs, discs, handpieces, excavators, turbines, files, reemers, (plastic) re-usables, disposables, prosthetics, implants, 3D printed implants, inserts, measuring devices, spreaders etc..

Optionally, the decontamination device is in accordance with norm EN ISO 15883-4 for washers-disinfectors. Optionally, the decontamination device is in accordance with norm EN ISO 14937 for sterilization of health care products. Optionally, the decontamination device is simultaneously in compliance with both norms.

The invention also relates to a system including a decontamination device as described above and at least one cassette arranged for holding the instrument. Optionally, the system includes a plurality of cassettes. It is possible that the system includes cassettes of a first type and cassettes of a second type. The cassettes of the fist type may be arranged for receiving instruments of a first type. The cassettes of the second type may be arranged for receiving instruments of a different second type. It is also possible that the cassettes of the first type are arranged for undergoing a first decontamination cycle and the cassettes of the second type are arranged for undergoing a different second decontamination cycle.

The cassettes of the first type may differ from the cassettes of the second type in one or more mechanical features. The cassettes of the first and the second type may differ in a marker present on or in the cassette. The marker can e.g. be a bar-code, QR-code, RFID device, NFID device, color, or the like. The decontamination device can be arranged to recognize the marker of the cassette and to perform a decontamination cycle according to the identified marker.

The invention also relates to a method for decontaminating a medical instrument using a decontamination device as defined in claim 12. The method includes receiving a to be treated medical instrument loaded in a cassette in a reception unit of the device, wherein the cassette includes the first portion and the second portion that can be closed against each other for enclosing the medical instrument. The method includes washing and/or drying the instrument in the cassette in a washing unit of the device. The method includes disinfecting and/or sterilizing the instrument in the cassette in a disinfection unit of the device. The method includes sealing the cassette containing the disinfected and/or sterilized instrument by a wrapping unit of the device. The method includes presenting the cassette containing the disinfected and/or sterilized instrument to be taken out of the decontamination device in a delivery unit of the device.

Optionally, the method includes providing the disinfected instrument in a holder, e.g. hermetically sealed from ambient air, by a wrapping unit. The step of presenting then includes presenting the, e.g. hermetically sealed, holder including the disinfected instrument to be taken out of the delivery unit.

Optionally, the decontamination device is a decontamination device as described above.

It will be appreciated that any of the features and options described in view of the device apply equally to the system and method, and vice versa. It will also be clear that any one or more of the above features and options can be combined.

### BRIEF DESCRIPTION OF THE DRAWINGS

Embodiments of the present invention will now be described in detail with reference to the accompanying drawings in which:
Figure 1 is a schematic representation of a decontamination device;
Figure 2 is a schematic representation of a cassette;
Figure 3 is a schematic representation of a decontamination device;
Figure 4 is a schematic representation of a decontamination device.

### DETAILED DESCRIPTION

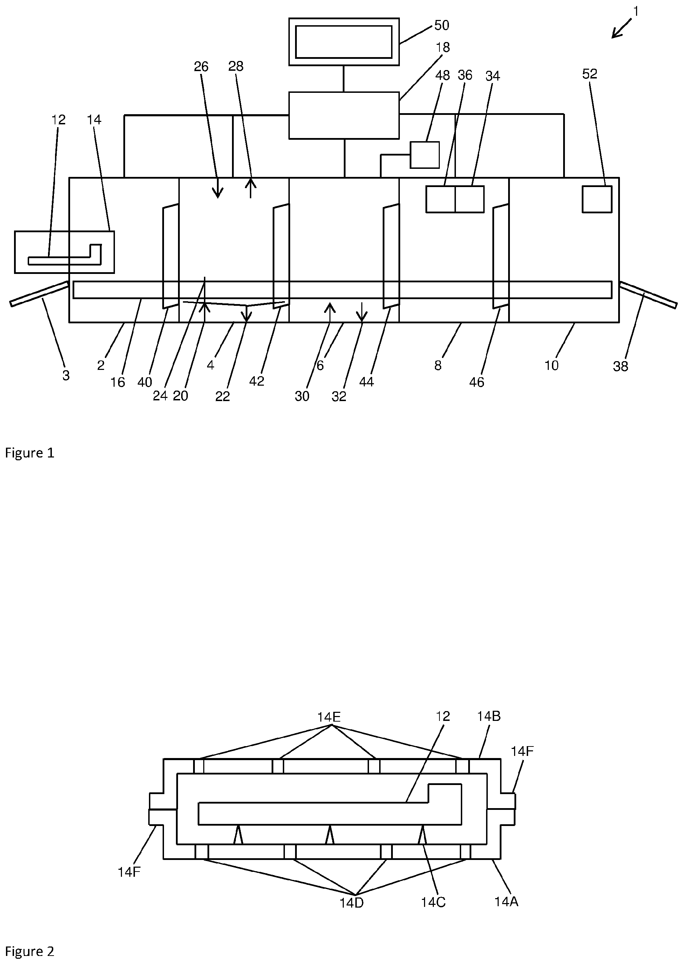

Figure 1 shows a schematic representation of a decontamination device 1 according to the invention. In this example the decontamination device includes a reception unit 2, a washing unit 4, a disinfection unit 6, a wrapping unit 8, and a delivery unit 10. The decontamination device 1 is arranged for decontaminating a medical instrument 12. Thereto, the medical instrument 12 to be treated is inserted into the reception unit 2, e.g. via a loading door 3. In Figure 1, the medical instrument 12 is loaded in a cassette 14. In this example, the device 2 further includes a transportation unit 16 for transporting the cassette 14 from unit to unit as described below. The to be decontaminated instrument 12 is loaded into the cassette 14. The cassette 14 is placed into the reception unit 2. In the reception unit 2 the cassette 14 is engaged by the transportation unit 16. The transportation unit 16 transports the cassette 14 from the reception unit 2 to the washing unit 4. In the washing unit 4 the instrument 12 can be washed. It will be appreciated that the decontamination device 1 may be operated according to different decontamination cycles. A control unit 18 is arranged for controlling operation of the respective units. The washing unit 4 can be controlled to perform different washing cycles. For example, as a first cycle the washing unit 4 may rinse the instrument 12 for washing off debris. Rinsing may e.g. be done using a liquid such as water. Thereto the washing unit 4 includes a washing liquid supply 20 and a washing liquid drain 22. For example as a second cycle the washing unit 4 may wash the instrument 12 for at least partly washing of contaminants. Washing may e.g. be done using a liquid such as water and/or a detergent. In this example the washing unit 4 includes one or more nozzles 24 for spraying a jet or mist of the liquid directly onto the instrument 12. For example as a third cycle the washing unit may disinfect the instrument 12 using a liquid, such as water, containing ozone as a disinfectant. It will be appreciated that the washing unit may use a warm or hot liquid. For example as a fourth cycle the washing unit 4 may dry the instrument 12 using a gas. Thereto the washing unit 4 includes a drying gas supply 26 and a drying gas exhaust 28. For example as a fifth cycle the washing unit 4 may disinfect the instrument 12 using a gas containing ozone. The control unit 18 controls the washing unit 4 for performing one of more of the first, second, third, fourth or fifth cycle, the order of the cycles, the duration of the cycles, the temperatures used in the cycles, etc..

The transportation unit 16 transports the cassette 14 from the washing unit 4 to the disinfection unit 6. The disinfection unit 6 is arranged for disinfecting the instrument 12. The disinfection unit 6 can be controlled to perform different disinfection cycles. For example as a first cycle the disinfection unit 6 may disinfect the instrument using a disinfectant, such as gaseous ozone. Thereto the disinfection unit includes a disinfectant supply 30 and a disinfectant drain 32. For example as a second cycle the disinfection unit 6 may sterilize the instrument using a disinfectant, such as gaseous ozone. It will be appreciated that other disinfectants may also be used such as steam, ethylene oxide gas, dry heat, hydrogen peroxide, peracetic acid, performic acid, phenolics, etc.. It will be appreciated that the disinfection unit 6 may also include a plurality of disinfectant supplies 30 for supplying a plurality of different disinfectants. This allows the user, or the control unit 18, to select one or more appropriate disinfectants for the decontamination cycle. It is for instance possible to first perform a disinfection cycle using gaseous ozone, followed by a sterilization cycle using steam. However, other combinations are also possible. The control unit 18 controls the disinfection unit 6 for performing one of more of the first or second cycles, the order of the cycles, the duration of the cycles, the temperatures used in the cycles, the concentrations of disinfectants used in the cycles etc..

The transportation unit 16 transports the cassette 14 from the disinfection unit 6 to the wrapping unit 8. In this example, the wrapping unit 8 is arranged for sealing the cassette 14. The wrapping unit 8 can be controlled to perform different disinfection cycles. For example as a first cycle the wrapping unit 8 may seal the cassette 14 with a tamper proof seal. The tamper proof seal e.g. needs to be broken to remove the disinfected instrument 12 from the cassette 14. The tamper proof seal thus provides a guarantee that the instrument 12 has not been touched or used since disinfection. Thereto the wrapping unit 8 includes a seal supply unit 34. The seal supply unit 34 may e.g. include a plurality of seals, such as self-adhesive stickers, tape or the like. For example as a second cycle the wrapping unit 8 may hermetically seal the cassette 8. For example as a third cycle the wrapping unit 8 may enclose the cassette 14, or the instrument 12 in case no cassette 14 is used, in a holder such as a bag, box, container or the like. Thereto the wrapping unit 8 may include a holder supply unit 36. The wrapping unit 8 may hermetically seal the holder after insertion of the cassette 14 or instrument 12. Thereto the wrapping unit 8 may provide a seal, such as a self-adhesive ribbon or glue, to the cassette. The wrapping unit 8 may e.g. apply the seal to a separation seam between the tray and the lid of the cassette 14. The wrapping unit 8 may also apply the seal to any openings in the cassette 14. The control unit 18 controls the wrapping unit 8 for performing one of more of the first, second or third cycles, the order of the cycles, etc..

The transportation unit 16 transports the cassette 14 from the wrapping unit 8 to the delivery unit 10. The delivery unit is arranged for presenting the disinfected instrument 12 in the cassette 14, to be taken out of the decontamination device 1. Thereto the delivery unit 10 may include an unloading door 38. The control unit 18 controls the delivery unit 10, e.g. for locking the unloading door 38 for preventing unauthorized opening at unwanted, e.g. hazardous, moments. It will be appreciated that the decontamination device uses disinfectants, possibly in gaseous form. The loading door 3 and unloading door 38 therefore may require lock-down during application of such disinfectant. It is also possible that the decontamination device includes one or more internal doors such as doors 40, 42, 44, 46 between the reception unit 2 and the washing unit 4, the washing unit 4 and the disinfection unit 6, the disinfection unit 6 and the wrapping unit 8, and the wrapping unit and the delivery unit 10, respectively. Lock-down of the internal doors 40, 42, 44, 46 can be controlled by the control unit 18. It is also possible that the decontamination device 1 includes a pump device 48 for maintaining the decontamination device 1, e.g. the disinfection unit 6, at an underpressure in order to avoid egress of gaseous disinfectants.

As can be seen from Figure 1, in this example the reception unit 2, especially the loading door 3 is physically separated from the delivery unit 10, especially the unloading door 38. There is a nonzero distance between the loading door 3 and the unloading door 38. Thus it is easy to keep contaminated instruments 12 away from decontaminated instruments 12. It is possible that the loading door 3 is in a first room and the unloading door 38 is in a different second room. In that case the decontamination device 1 extends through a wall between the first and second rooms. Hence, the first room can be a contaminated room, while the second room is a decontaminated room.

The decontamination device 1 performs the decontamination cycle under control of the control unit 18. The decontamination cycle can be selected and/or configured using user interface 50. The user interface may include one or more buttons, controls, a display, and/or a touchscreen. It will be appreciated that the control unit 18 controls the reception unit 2, the washing unit 4, the disinfection unit 6, the wrapping unit 8 and the delivery unit 10 for performing one of more of the respective cycles. It will be appreciated that it is also possible that the control unit 18 configures a decontamination cycle in which one or more of the washing unit 4, the disinfection unit 6 and the wrapping unit 8 does not perform a cycle.

In the example of Figure 1 the decontamination device 1 includes an optional labelling unit 52. The labelling unit 52 is arranged for providing the instrument 12 and/or the cassette 14 with a label representative of the performed decontamination. The label can e.g. be a sticker or can be printed onto the instrument 12 and/or the cassette 14. Hence it is possible to determine the decontamination performed on the instrument from the label. The label can include information on the performed decontamination, e.g. a code representative of the performed decontamination cycle. The label can also include information representative of a location in a database where information on the performed decontamination cycle is stored. The label can include information representative of a date and time of the performed decontamination cycle.

Figure 2 shows a schematic representation of a cassette 14. In this example the cassette includes a tray 14A and a lid 14B. In this example the tray 14A includes carriers 14C for carrying an instrument 12. In this example the tray 14A includes openings 14D for allowing washing liquids, drying gases and disinfectants to pass. In this example the lid 14B includes openings 1E for allowing washing liquids, drying gases and disinfectants to pass. Preferably the openings 14D, 14E are arranged such that they can be easily sealed by the wrapping unit 8. Thereto, the openings may e.g. be positioned close to the interface between the tray 14A and the lid 14B. In Figure 2 the cassette 14 includes handling points 14F arranged for being engaged by the transportation unit 16.

It will be appreciated that different types of cassettes 14 may be associated with the decontamination device 1. There may be cassettes 14 of a first type and cassettes 14 of a second type. The cassettes 14 of the fist type may be arranged for receiving instruments 12 of a first type. The cassettes 14 of the second type may be arranged for receiving instruments 12 of a different second type. Thereto the cassettes of the first and second types may include mutually different carriers 14C. It is possible that the exterior dimensions of the cassettes of the first and second type are the same. It is also possible that the exterior dimensions of the cassettes of the first and the second type are different. In the latter case preferably the handling points 14F of both types of cassettes are the same, so that the transportation unit 16 can handle both types of cassettes.

It is also possible that the cassettes 14 of the first type are arranged for undergoing a first decontamination cycle and the cassettes 14 of the second type are arranged for undergoing a different second decontamination cycle. Thereto the user may instruct the control unit 18 to perform the different decontamination cycles.

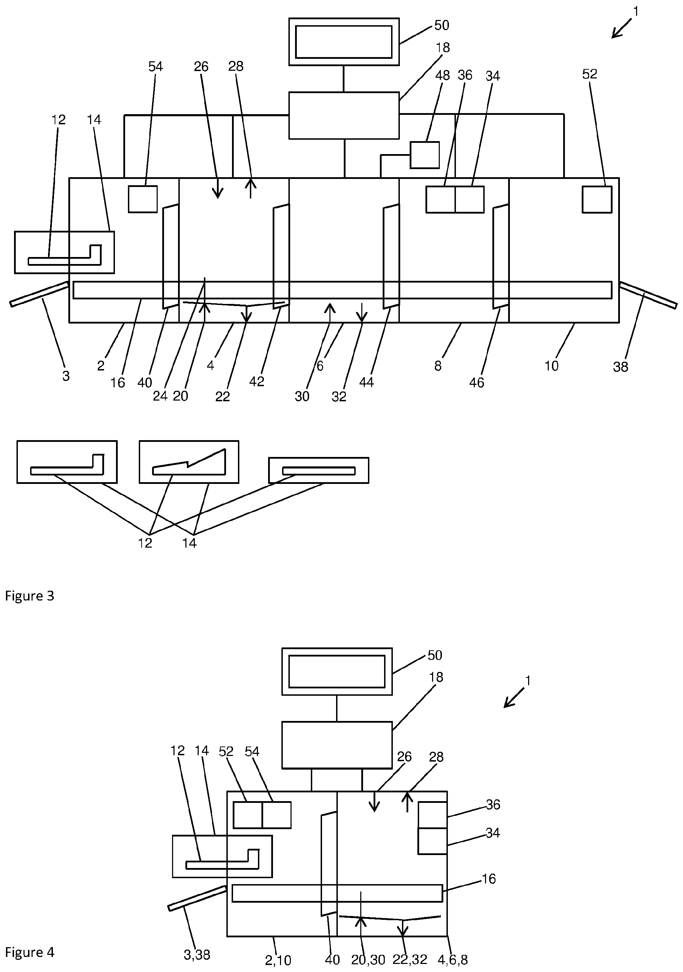

Figure 3 shows a schematic representation of a decontamination device 1 according to the invention. This decontamination device is similar to the decontamination device 1 shown in Figure 1. In this example, the decontamination device 2 further includes a recognition unit 54. The recognition unit 54 is arranged for recognizing the different types of cassettes. It is possible that the control unit 18 stores a plurality of sets of instructions, each representative of a specific decontamination cycle. The control unit 18 may store a list including for each type of cassette an associated decontamination cycle. Upon recognizing the type of cassette, the decontamination device 2 can automatically select the associated decontamination cycle, and perform said cycle. Hence, medical instruments can easily be loaded into an appropriate cassette that has a decontamination cycle associated therewith that is suitable for the instrument at hand.

The different types of cassettes may differ in a marker present on or in the respective cassettes. The marker can e.g. be a mechanical indicator, a bar-code, a two-dimensional barcode, a QR-code, a Radio Frequency Identification (RFID) device, a Near Field Identification (NFID) device, a color of the cassette, a color of part of the cassette, a color code or the like. The recognition unit 54 decontamination device can be arranged to recognize the marker of the cassette and to perform a selected decontamination cycle according to the identified marker.

Figure 4 shows a schematic representation of a decontamination device 1 according to the invention. The functioning of this decontamination device 1 is similar to that of the device 1 described with respect to Figure 1 and Figure 3. In this example, the reception unit 2 and the delivery unit share the same space. In this example, one physical entity performs both the function of the reception unit 2 and the delivery unit 10. In this example the joint reception and delivery unit includes a single loading and unloading door. In this example, the washing unit 4, the disinfection unit 6 and the wrapping unit 8 share the same space. In this example one physical entity performs the functions of the washing unit 4, the disinfection unit 6 and the wrapping unit 10. It will be clear that the decontamination device may include an internal door 40. The decontamination device of the present example can for instance be embodied as a desk top or counter top device. The decontamination device may be embodied in a size according to general kitchen appliances, such as an oven or microwave oven. The dimensions of the decontamination device may be approximately 60cm wide, 40cm high and 50cm deep. Hence, the decontamination device can easily be integrated into a medical work environment, such as a cupboard e.g. of a dental practice.

Herein, the invention is described with reference to specific examples of embodiments of the invention. It will, however, be evident that various modifications and changes may be made therein, without departing from the essence of the invention as defined in the appended claims. For the purpose of clarity and a concise description features are described herein as part of the same or separate embodiments, however, alternative embodiments having combinations of all or some of the features described in these separate embodiments are also envisaged.

In the example of Figure 1 the reception unit 2, the washing unit 4, the disinfection unit 6, the wrapping unit 8 and the delivery unit 10 are shown as physically separate units occupying different spaces. It will be appreciated that one or more of the reception unit 2, the washing unit 4, the disinfection unit 6, the wrapping unit 8 and the delivery unit 10 may also share the same space. It is for instance possible that the reception unit 2 occupies the same space as the washing unit 4, i.e. for instance the washing cycles being performed on the cassette 14 or instrument 12 on the same location where it was inserted into the decontamination device 1. It will be appreciated that the transportation unit 16 then need not transport the cassette 14 or instrument 12 from the reception unit 2 to the washing unit 4. Similarly, the washing unit 4 and the disinfection unit 6 can occupy the same space, the disinfection being performed on the cassette 14 or instrument 12 at the same location as the washing. Similarly, the disinfection unit 6 and the wrapping unit 8 can occupy the same space, the wrapping being performed on the cassette 14 or instrument 12 at the same location as the disinfecting. Similarly, the wrapping unit 8 and the delivery unit 10 can occupy the same space, the presenting of the cassette 14 or instrument 12 being performed at the same location as the wrapping.

In the examples mention is made of cassettes of a first type and cassettes of a second type. It will be appreciated that it is possible that the decontamination device has a plurality of types of cassettes associated therewith.

In the examples, the disinfection unit uses ozone and/or another disinfectant for disinfecting and/or sterilizing the instrument. It will be appreciated that the washing unit may also use ozone and/or another disinfectant while washing and/or drying the instrument.

In the examples is referred to dental instruments. It will be appreciated that the to be decontaminated medical instruments can also be instruments or implants for use in other medical fields, such as general medicine, dental medicine, ocular medicine, veterinary medicine, bowel and intestinal medicine, cardio medicine, cardio vascular medicine, oncology medicine, ophthalmology, gynecology, endodontology, etc.. The decontamination device can also be used for flash sterilization e.g. in operating rooms. It will be appreciated that the medical instruments to be decontaminated can also be instruments used in a medical environment, such as wearables at a point of care, chairside or bedside. Examples of such wearables are for instance cellphones, beepers, stethoscopes, pens, etc.. It will be appreciated that the control unit can be embodied as dedicated electronic circuits, possibly including software code portions. The control unit can also be embodied as software code portions executed on, and e.g. stored in, a memory of, a programmable apparatus such as a computer.

Although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal which may be conveyed via electrical or optical cable or by radio or other means, e.g. via the internet or cloud.

When a program is embodied in a signal which may be conveyed directly by a cable or other device or means, the carrier may be constituted by such cable or other device or means. Alternatively, the carrier may be an integrated circuit in which the program is embedded, the integrated circuit being adapted for performing, or for use in the performance of, the relevant processes.

However, other modifications, variations, and alternatives are also possible. The specifications, drawings and examples are, accordingly, to be regarded in an illustrative sense rather than in a restrictive sense.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to an advantage.

## Claims

1. A decontamination device (1) for medical instruments (12) including within a single device:
a reception unit (2) arranged for receiving a to be treated medical instrument (12) loaded in a cassette (14) wherein the cassette includes a first portion and a second portion that can be closed against each other for enclosing the medical instrument;
a washing unit (4) arranged for washing and/or drying the instrument (12) in the cassette;
a disinfection unit (6) arranged for disinfecting and/or sterilizing the instrument in the cassette;
a wrapping unit (8) arranged for sealing the cassette (14) containing the disinfected and/or sterilized instrument; and
a delivery unit (10) arranged for presenting the cassette (14) containing the disinfected and/or sterilized instrument (12) to be taken out of the decontamination device.

2. The decontamination device (1) of claim 1, wherein the washing unit (4) and the disinfection unit (6) occupy the same space.

3. The decontamination device (1) of any one of claims 1-2, including a control unit (18) arranged for controlling the washing unit (4) and the disinfection unit (6), for automatically washing and/or drying and disinfecting the instrument.

4. The decontamination device (1) of claim 3, wherein the disinfection unit (6) is arranged to be controlled to in a first cycle disinfect the instrument and in a second cycle sterilize the instrument.

5. The decontamination device (1) of any one of claims 1-4, wherein the disinfection unit (6) is arranged for disinfecting and/or sterilizing the instrument with ozone.

6. The decontamination device (1) of any one of claims 1-5, wherein the washing unit (4) is arranged for washing debris off the instrument.

7. The decontamination device (1) of claim 6, wherein the washing unit (4) includes at least one nozzle (24) to spray a jet or mist of a cleaning fluid directly onto the instrument, and/or to spray a jet or mist of a drying gas directly onto the instrument.

8. The decontamination device (1) of any one of claims 1-7, including a labelling unit (52) arranged for providing the instrument and/or the cassette with a label representative of the performed decontamination.

9. The decontamination device of any one of claims 1-8, wherein the instrument is a dental instrument.

10. The decontamination device (1) of any one of claims 1-9, wherein the device is a desk top or counter top device.

11. System including a decontamination device (1) of any one of claims 1-10 and at least one cassette (14) arranged for holding the instrument.

12. A method for decontaminating a medical instrument (12) using a decontamination device (1), the method including:
receiving a to be treated medical instrument (12) loaded in a cassette (14) in a reception unit (2) of the device (1), wherein the cassette includes a first portion and a second portion that can be closed against each other for enclosing the medical instrument;
washing and/or drying the instrument in the cassette in a washing unit (4) of the device;
disinfecting and/or sterilizing the instrument in the cassette in a disinfection unit (6) of the device;
sealing the cassette (14) containing the disinfected and/or sterilized instrument (12) by a wrapping unit (8) of the device; and
presenting the cassette containing the disinfected and/or sterilized instrument to be taken out of the decontamination device in a delivery unit (10) of the device.

13. The method of claim 12, wherein the decontamination device is a decontamination device according to any one of claims 1-11.

## Patentansprüche

1. Dekontaminationsvorrichtung (1) für medizinische Instrumente (12), die Folgendes innerhalb einer einzigen Vorrichtung beinhaltet:
eine Aufnahmeeinheit (2), die zum Aufnehmen eines zu behandelnden medizinischen Instruments (12) eingerichtet ist, das in eine Kassette (14) geladen ist, wobei die Kassette einen ersten Abschnitt und einen zweiten Abschnitt beinhaltet, die gegeneinander zum Umschließen des medizinischen Instruments geschlossen werden können;
eine Wascheinheit (4), die zum Waschen und/oder Trocknen des Instruments (12) in der Kassette eingerichtet ist;
eine Desinfektionseinheit (6), die zum Desinfizieren und/oder Sterilisieren des Instruments in der Kassette eingerichtet ist;
eine Wickeleinheit (8), die zum Abdichten der Kassette (14), die das desinfizierte und/oder sterilisierte Instrument enthält, eingerichtet ist;
eine Liefereinheit (10), die zum Präsentieren der Kassette (14), die das desinfizierte und/oder sterilisierte Instrument (12) enthält, zum Herausnehmen aus der Dekontaminationsvorrichtung eingerichtet ist.

2. Dekontaminationsvorrichtung (1) nach Anspruch 1, wobei die Wascheinheit (4) und die Desinfektionseinheit (6) denselben Raum einnehmen.

3. Dekontaminationsvorrichtung (1) nach einem der Ansprüche 1 bis 2, die eine Steuereinheit (18) beinhaltet, die zum Steuern der Wascheinheit (4) und der Desinfektionseinheit (6) eingerichtet ist, um das Instrument automatisch zu waschen und/oder zu trocknen und zu desinfizieren.

4. Dekontaminationsvorrichtung (1) nach Anspruch 3, wobei die Desinfektionseinheit (6) dazu eingerichtet ist, gesteuert zu werden, um in einem ersten Zyklus das Instrument zu desinfizieren und in einem zweiten Zyklus das Instrument zu sterilisieren.

5. Dekontaminationsvorrichtung (1) nach einem der Ansprüche 1 bis 4, wobei die Desinfektionseinheit (6) zum Desinfizieren und/oder Sterilisieren des Instruments mit Ozon eingerichtet ist.

6. Dekontaminationsvorrichtung (1) nach einem der Ansprüche 1 bis 5, wobei die Wascheinheit (4) zum Abwaschen von Rückständen von dem Instrument eingerichtet ist.

7. Dekontaminationsvorrichtung (1) nach Anspruch 6, wobei die Wascheinheit (4) mindestens eine Düse (24) zum Sprühen eines Strahls oder Nebels eines Reinigungsfluids direkt auf das Instrument, und/oder zum Sprühen eines Strahls oder Nebels eines Trocknungsgases direkt auf das Instrument beinhaltet.

8. Dekontaminationsvorrichtung (1) nach einem der Ansprüche 1 bis 7, die eine Etikettierungseinheit (52) beinhaltet, die zum Versehen des Instruments und/oder der Kassette mit einem Etikett, das die durchgeführte Dekontamination darstellt, eingerichtet ist.

9. Dekontaminationsvorrichtung nach einem der Ansprüche 1 bis 8, wobei das Instrument ein Dentalinstrument ist.

10. Dekontaminationsvorrichtung (1) nach einem der Ansprüche 1 bis 9, wobei die Vorrichtung eine Tisch- oder Arbeitsplattenvorrichtung ist.

11. System, das eine Dekontaminationsvorrichtung (1) nach einem der Ansprüche 1 bis 10 und mindestens eine Kassette (14), die zum Halten des Instruments eingerichtet ist, beinhaltet.

12. Verfahren zum Dekontaminieren eines medizinischen Instruments (12) unter Verwendung einer Dekontaminationsvorrichtung (1), wobei das Verfahren Folgendes beinhaltet:
Aufnehmen eines zu behandelnden medizinischen Instruments (12), das in eine Kassette (14) geladen ist, in einer Aufnahmeeinheit (2) der Vorrichtung (1), wobei die Kassette einen ersten Abschnitt und einen zweiten Abschnitt beinhaltet, die gegeneinander zum Umschließen des medizinischen Instruments geschlossen werden können;
Waschen und/oder Trocknen des Instruments in der Kassette in einer Wascheinheit (4) der Vorrichtung;
Desinfizieren und/oder Sterilisieren des Instruments in der Kassette in einer Desinfektionseinheit (6) der Vorrichtung;
Abdichten der Kassette (14), die das desinfizierte und/oder sterilisierte Instrument (12) enthält, durch eine Wickeleinheit (8) der Vorrichtung; und
Präsentieren der Kassette, die das desinfizierte und/oder sterilisierte Instrument enthält, in einer Liefereinheit (10) der Vorrichtung, um aus der Dekontaminationsvorrichtung herausgenommen zu werden.

13. Verfahren nach Anspruch 12, wobei die Dekontaminationsvorrichtung eine Dekontaminationsvorrichtung nach einem der Ansprüche 1 bis 11 ist.

## Revendications

1. Dispositif de décontamination (1) pour instruments médicaux (12) comprenant dans un seul dispositif :
une unité de réception (2) agencée pour recevoir un instrument médical à traiter (12) chargé dans une cassette (14), dans lequel la cassette comprend une première partie et une deuxième partie qui peuvent être fermées l'une contre l'autre pour enfermer l'instrument médical ;
une unité de lavage (4) agencée pour laver et/ou sécher l'instrument (12) dans la cassette ;
une unité de désinfection (6) agencée pour désinfecter et/ou stériliser l'instrument dans la cassette ;
une unité d'emballage (8) agencée pour sceller la cassette (14) contenant l'instrument désinfecté et/ou stérilisé ; et
une unité de distribution (10) agencée pour présenter la cassette (14) contenant l'instrument désinfecté et/ou stérilisé (12) devant être retiré du dispositif de décontamination.

2. Dispositif de décontamination (1) selon la revendication 1, dans lequel l'unité de lavage (4) et l'unité de désinfection (6) occupent le même espace.

3. Dispositif de décontamination (1) selon l'une quelconque des revendications 1 à 2, comprenant une unité de commande (18) agencée pour commander l'unité de lavage (4) et l'unité de désinfection (6) afin de laver et/ou sécher et désinfecter automatiquement l'instrument.

4. Dispositif de décontamination (1) selon la revendication 3, dans lequel l'unité de désinfection (6) est agencée pour être commandée dans un premier cycle qui désinfecte l'instrument et dans un deuxième cycle qui stérilise l'instrument.

5. Dispositif de décontamination (1) selon l'une quelconque des revendications 1 à 4, dans lequel l'unité de désinfection (6) est agencée pour désinfecter et/ou stériliser l'instrument avec de l'ozone.

6. Dispositif de décontamination (1) selon l'une quelconque des revendications 1 à 5, dans lequel l'unité de lavage (4) est agencée pour éliminer les débris de l'instrument, par lavage.

7. Dispositif de décontamination (1) selon la revendication 6, dans lequel l'unité de lavage (4) comprend au moins une buse (24) pour pulvériser un jet ou un brouillard d'un fluide de nettoyage directement sur l'instrument, et/ou pour pulvériser un jet ou un brouillard d'un gaz de séchage directement sur l'instrument.

8. Dispositif de décontamination (1) selon l'une quelconque des revendications 1 à 7, comprenant une unité d'étiquetage (52) agencée pour doter l'instrument et/ou la cassette d'une étiquette représentative de la décontamination réalisée.

9. Dispositif de décontamination (1) selon l'une quelconque des revendications 1 à 8, dans lequel l'instrument est un instrument dentaire.

10. Dispositif de décontamination (1) selon l'une quelconque des revendications 1 à 9, dans lequel le dispositif est un dispositif conçu pour être posé sur une table ou une paillasse.

11. Système comprenant un dispositif de décontamination (1) selon l'une quelconque des revendications 1 à 10 et au moins une cassette (14) agencée pour maintenir l'instrument.

12. Procédé pour décontaminer un instrument médical (12) en utilisant un dispositif de décontamination (1), le procédé comprenant les faits de :
recevoir un instrument médical à traiter (12) chargé dans une cassette (14) dans une unité de réception (2) du dispositif (1), dans lequel la cassette comprend une première partie et une deuxième partie qui peuvent être fermées l'une contre l'autre pour enfermer l'instrument médical ;
laver et/ou sécher l'instrument dans la cassette dans une unité de lavage (4) du dispositif ;
désinfecter et/ou stériliser l'instrument dans la cassette dans une unité de désinfection (6) du dispositif ;
sceller la cassette (14) contenant l'instrument désinfecté et/ou stérilisé (12) avec une unité d'emballage (8) du dispositif ; et
présenter la cassette contenant l'instrument désinfecté et/ou stérilisé devant être retiré du dispositif de décontamination dans une unité de distribution (10) du dispositif.

13. Procédé selon la revendication 12, dans lequel le dispositif de décontamination est un dispositif de décontamination selon l'une quelconque des revendications 1 à 11.
